# EUROPÄISCHE PATENTANMELDUNG

(11) **EP 2 087 901 A1**
(43) Veröffentlichungstag der Anmeldung: **12.08.2009**
(21) Anmeldenummer: 08151198.2
(22) Anmeldetag: 08.02.2008
(51) Int. Cl.: A61K 36/28

(54) **Verfahren zu Gewinnen von Edelweißinhaltsstoffen, ihre Verwendung und Zusammensetzung**

(71) Anmelder: Morgenstern, Claus, 22391 Hamburg (DE)
(72) Erfinder: Morgenstern, Claus, 22391 Hamburg (DE)
(74) Vertreter: Raffay & Fleck

(57) **Zusammenfassung**

Die Erfindung betrifft ein Verfahren zum Gewinnen von Edelweißinhaltsstoffen, ihre Verwendung und Zusammensetzung. Bekanntermaßen gibt es unter den Edelweißarten ca. 30 Unterarten. Zumindest in Europa stehen diese unter Artenschutz und werden deshalb gezüchtet. Die daraus gewonnenen Inhaltsstoffe sind nicht nur äußerst teuer, sondern auch überwiegend instabil. Hier schafft die Erfindung Abhilfe durch ein Verfahren mit folgenden Schritten:
a. Ernten der asiatischen Edelweißarten Leontopodium ochroleuceum und/oder Leontopodium leontopoides und/oder Leontopodium himalayanum mit Blättern, Blüte, Stengel und/oder Wurzel,
b. anschließendes Trocknen und Gefriertrocknen und Verarbeiten zu Pulver,
c. Extrahieren des entstandenen Pulvers mit einer alkoholischen Lösung und Schütteln derselben für eine Vielzahl von Stunden, insbesondere 24h,
d. Abtrennen der Feststoffe und ggf. Einengen der extrahierten alkoholischen Lösung,
e. Stabilisieren der Lösung mittels einem Paraben enthaltenden Konservierungsstoff und ggf. vollständiges Entfernen des Alkohols.

Die erfindungsgemäße Zusammensetzung ist besonders zur dentalen Pflege geeignet (Fig. 2).

## Beschreibung

### Technisches Gebiet

Die Erfindung betrifft ein Verfahren zum Gewinnen von Edelweißinhaltsstoffen, ihre Verwendung und Zusammensetzung.

### Stand der Technik

Das Edelweiß-Leontopodium (Löwenfuß) gehört zur Familie der Korbblüter (Asteraceen). Es wächst auf kalkigen Böden in einer Höhe von 1500 m bis 3400 m. Die Pflanze ist aufgrund ihres Wachstums starker UV-Strahlung, einem niedrigen Luftdruck und extremen Temperatur- und Feuchtigkeitsschwankungen ausgesetzt. Entwicklungsgeschichtlich bildete die Pflanze Schutzfaktoren, welche von der Natur im Laufe der Jahrtausende optimiert wurden.

Diese pflanzlichen Inhaltsstoffe schützen bekanntermaßen auch unsere Haut vor widrigen Umwelteinflüssen. Unter den Edelweißarten gibt es ca. 30 Unterarten.

Auf dem Markt sind die unterschiedlichsten Produkte mit Edelweißinhaltsstoffen bekannt, ebenso wie ihr Einsatz in der Kosmetik, insbesondere als Sonnencreme wegen ihrer antioxidativen Eigenschaften aufgrund der Inhaltsstoffe (Chlorogensäure, Luteolin, Apigenin-7-Glucosid, β-Sitosterol). Daneben ist eine direkte enzymhemmende Eigenschaft bekannt, die Hyaluronidase, 5-Lipoxygenase und Elastase hemmen. Es wird zur Behandlung empfindlicher und gereizter Haut im Sinne eines Antiaging- und Aftersun-Präparates angewandt. Insbesondere hat man sich mit dem unter Artenschutz stehenden Leontopodium alpinum beschäftigt, das in Europa ansässig ist und in Form von gezüchteten Wiesenpflanzen im Wallis gewonnen wird. Die so hergestellten Mengen der Inhaltsstoffe des Alpinums sind verständlicherweise äußerst beschränkt und deswegen teuer. Zudem ist die Instabilität der Inhaltsstoffe ein immer wiederkehrendes Problem.

### Darstellung der Erfindung

Der Erfindung liegt deshalb die Aufgabe zugrunde, ein Verfahren zum Gewinnen von Edelweißinhaltsstoffen zu schaffen, das äußerst wirtschaftlich ist und zu einer neuen, stabilen und preiswerten Zusammensetzung führt, die - wenn möglich - einen zusätzlichen Verwendungszweck besitzt.

Diese Aufgabe wird in überraschenderweise durch ein Verfahren mit folgenden Schritten gelöst:

a. Ernten der asiatischen Edelweißarten Leontopodium ochroleuceum und/oder Leontopodium leontopoides und/oder Leontopodium himalayanum mit Blättern, Blüte, Stengel und/oder Wurzel,

b. anschließendes Trocknen und Gefriertrocknen und Verarbeiten zu Pulver,

c. Extrahieren des entstandenen Pulvers mit einer alkoholischen Lösung und Schütteln derselben für eine Vielzahl von Stunden, insbesondere 24h,

d. Abtrennen der Feststoffe und ggf. Einengen der extrahierten alkoholischen Lösung,

e. Stabilisieren der Lösung mittels einem Paraben enthaltenden Konservierungsstoff und ggf. vollständiges Entfernen des Alkohols.

Von besonderer erfindungsgemäßer Bedeutung ist die Tatsache, dass ausschließlich asiatische Edelweiße, nämlich das Leontopodium ochroleuceum und das Leontopodium leontopoides bzw. das Leontopodium himalayanum in Asien gesammelt bzw. geerntet werden. Untersuchungen mit HPC-Analyse haben gezeigt, dass gerade diese Unterarten des Leontopodiums zusätzliche Wirkstoffe gegenüber dem Alpinum enthalten. Des weiteren hat sich gezeigt, dass das an die Ernte sich anschließende Trocknen und Gefriertrocknen und Verarbeiten zu Pulver zu einem transportfähigem Zwischenprodukt nach den Schritten a und b führt, das seine Eigenschaften beibehält ohne nennenswerte Zerfallserscheinungen zu zeigen. Somit ist es möglich in der Mongolei zu ernten, trocknen und gefriertrocknen, so dass das gewonnene Pulver dann problemlos, ggf. per Flugzeug, nach Europa geschafft werden kann, wo eine bessere Aufarbeitung gemäß der Schritte c - e erfolgt. Der wichtigste Aspekt der Erfindung liegt jedoch in der Schaffung einer stabilen Lösung der Edelweißinhaltsstoffe, nämlich mit einem parabenenthaltenden Konservierungsstoff, vorteilhafter Weise in äthanolischer Lösung, so dass auch nach mehr als zwei Jahren die Wirkstoffe im Extrakt nachgewiesen werden können, ohne dass Farbänderungen oder sonstige Änderungen bei der HPLC-Analyse auftreten, wenn man einmal von den unterschiedlichen Intensitäten der Peaks absieht.

In überraschender Weise hat sich herausgestellt, dass eine erfindungsgemäße flüssige Zusammensetzung in Form einer stabilen alkoholischen Lösung zur dentalen Pflege eingesetzt werden kann, um insbesondere eine Weißung der menschlichen Zähne zu erzielen.

Es dürfte einleuchten, dass man in der Stufe e letztendlich auch eine vollständige Entfernung des Alkohols, insbesondere des Äthanols, vorsehen kann, um den Edelweißwirkstoff dann in der gewünschten galenischen Form zum Einsatz bringen zu können, so z.B. als Milch, Creme, Öl, die dann im kosmetischen Bereich als Sonnenschutzmittel oder Anti-aging-Mittel zum Einsatz kommt. Es dürfte einleuchten, dass sämtliche bekannten Verwendungszwecke hiervon umschlossen sind.

Die erfindungsgemäße Zusammensetzung enthält eine stabile Lösung oder ein gefriergetrocknetes Pulver von Edelweißextrakten der Spezies Leontopodium ochroleuceum und/oder Leontopodium leontopoides und/oder Leontopodium himalayanum, wobei sich zumindest bei der HPLC-Analyse zahlreiche Wirkstoffe in Form von Peaks gezeigt haben.

Weitere Vorteile und Merkmale gehen aus den Unteransprüchen hervor, die auch gemeinsam mit dem Hauptanspruch von erfinderischer Bedeutung sein können.

**Kurze Beschreibung der Abbildungen der Zeichnungen**

Weitere Vorteile und Merkmale der Erfindung ergeben sich aus der nachfolgenden Beschreibung eines Ausführungsbeispiels unter vergleichender Bezugnahme auf ein Beispiel aus dem Stand der Technik. Dabei zeigen:

Fig. 1 ein UV-Chromatogramm einer Probe nach dem Stand der Technik, enthaltend Leontopodium alpinum (Präparat der Marke Alpaflor®);

Fig. 2 ein UV-Chromatogramm einer erfindungsgemäßen Zusammensetzung.

### Weg(e) zur Ausführung der Erfindung

Im Folgenden wird ein bevorzugtes Ausführungsbeispiel zum besseren Verständnis der Erfindung gezeigt, auf das die Erfindung jedoch nicht beschränkt ist.

Als Untersuchungsmaterial wurde Edelweißdroge mit Blättern, Blüte und Stengel, jedoch ohne Wurzel der drei asiatischen Edelweißarten eingesetzt, die den Verfahrensschritten a und b des Anspruchs 1 unterzogen wurden, und zwar in einem Verhältnis von einem Teil Droge bzw. Pulver und 20 Teile Lösungsmittel (70% Äthanol in Wasser) zugefügt. Es wurde über 24 Stunden in der Schüttelmaschine bei Raumtemperatur extrahiert und ein gelbgrünlicher Extrakt erhalten, der gravimetrisch bestimmt wurde und einen Gehalt an extrahierbaren Substanzen von 10,7% ergab.

Weitere Versuche - auch mit einzelnen der drei genannten Edelweißarten führten zu ähnlichen Ergebnissen mit Ausbeuten im Bereich von 8 - 16%, was als äußerst gut anzusehen ist.

Die Analyse bezüglich der Farbe (gelblich mit leichtem Grünanteil) und des chromatografischen Profils aus dem getrockneten Edelweiß ergab identische Werte mit den 7 Monate gelagerten bzw. dem frischen Extrakt. Lediglich die Peak-Intensität war bei dem über längere Zeit getrockneten Material geringer als bei dem frisch getrockneten Edelweiß, weshalb Letzteres im Einsatz bevorzugt wird.

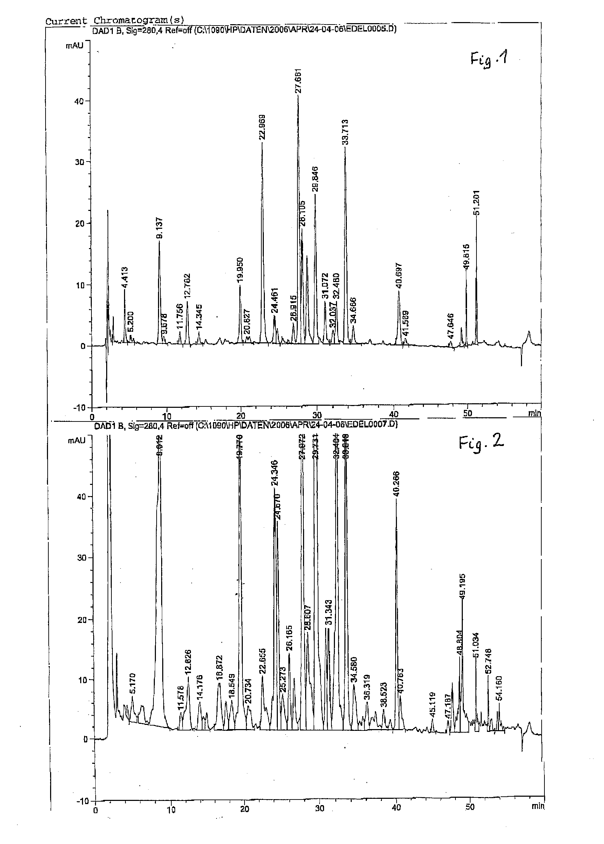

Chromatographie-UV-Absorptionspektren der erfindungsgemäßen 70%igen äthanolischen Zusammensetzung sehen nach 24 Stunden Extraktion derart aus, wie es in Fig. 2 dargestellt ist.

In der grafischen Darstellung ist das Absorptionsausmaß (mAU) gegen die Retentionszeit (min) aufgetragen.

Ein entsprechendes UV-Absorptionsspektrum von Leontopodium alpinum (Alpaflor ®) ist in Fig. 1 abgebildet. Es bestehen weitgehende Übereinstimmungen mit dem UV/-Spektrum der Fig. 2, wobei die erfindungsgemäße Zusammensetzung zusätzliche peaks aufweist, die das Vorhandensein weiterer Wirkstoffe anzeigen. Auch ist die Intensität zahlreicher peaks unterschiedlich.

Grob zuordnen kann man die peaks etwa wie folgt:

∼ 9 Chlorogensäure

- 14, 29, 32, 34 Chlorogensäurederivate

∼ 24 Luteolin-7-glykosid

- 29,5 Apigenin-7-glykosid

- 30 Luteolin-4-glykosid

- 37 Luteolin

Die mit Methyl- und/oder Propylparaben dotierten erfindungsgemäßen Edelweiß-Zusammensetzungen zeigen entsprechende zusätzliche peaks für diese Substanzen bei 27,76 bzw. 49.83 (nicht gezeigt).

## Patentansprüche

1. Verfahren zum Gewinnen von Edelweiß-Inhaltsstoffen, **gekennzeichnet durch** folgende Schritte:
a. Ernten der asiatischen Edelweißarten Leontopodium ochroleuceum und/oder Leontopodium leontopoides und/oder Leontopodium himalayanum mit Blättern, Blüte, Stengel und/oder Wurzel,
b. anschließendes Trocknen und Gefriertrocknen und Verarbeiten zu Pulver,
c. Extrahieren des entstandenen Pulvers mit einer alkoholischen Lösung und Schütteln derselben für eine Vielzahl von Stunden, insbesondere 24h,
d. Abtrennen der Feststoffe und ggf. Einengen der extrahierten alkoholischen Lösung,
e. Stabilisieren der Lösung mittels eines Paraben enthaltenden
Konservierungsstoff und ggf. vollständiges Entfernen des Alkohols.

2. Verfahren nach Anspruch 1, **dadurch gekennzeichnet, dass** die Extraktion gemäß Schritt c. mittels 60-80, insbesondere 70 prozentigen unvergälltem Äthanol bei Raumtemperatur durchgeführt wird.

3. Verfahren nach Anspruch 2, **dadurch gekennzeichnet, dass** 1 g gefriergetrocknetes Pulver mit 15-25 g 70%igem Äthanol extrahiert wird.

4. Verfahren nach Anspruch 1 bis 3, **dadurch gekennzeichnet, dass** gemäß Schritt e. Konservierungsstoffe Methyl- und/oder Propylparaben (4-Hydroxybenzolsäureester) und/oder 2-Oxyäthanol und/oder Ascorbinsäure und/oder Ascorbinpalmitat zugesetzt werden.

5. Verwendung der nach einem oder mehreren der Ansprüche 1 bis 4 hergestellten Lösung oder daraus hergestellten anderen Applikationsformen zur dentalen Pflege, insbesondere Weißung der menschlichen Zähne.

6. Zusammensetzung, die eine stabile Lösung oder Pulver von Edelweiß-Extrakt(en) der Spezies Leontopodium ochroleuceum und/oder Leontopodium leontopoides und/oder Leontopodium himalayanum enthält.
